(19) Europäisches Patentamt
European Patent Office
Office européen des brevets

(11) EP 0 767 684 B1

(12) EUROPEAN PATENT SPECIFICATION

(45) Date of publication and mention
of the grant of the patent:
**25.05.2005 Bulletin 2005/21**

(51) Int Cl.⁷: **A61M 29/00**, A61F 2/06,
B29C 67/02, B29D 23/00

(21) Application number: **95923776.9**

(22) Date of filing: **07.06.1995**

(86) International application number:
**PCT/US1995/007326**

(87) International publication number:
**WO 1996/000103 (04.01.1996 Gazette 1996/02)**

(54) **RADIALLY EXPANDABLE POLYTETRAFLUOROETHYLENE AND EXPANDABLE ENDOVASCULAR STENTS FORMED THEREWITH**

RADIAL EXPANDIERBARES POLYTETRAFLUORETHYLEN UND DARAUS GEFORMTE EXPANDIERBARE ENDOVASKULÄRE STENTS

POLYTETRAFLUOROETHYLENE EXPANSE RADIALEMENT, ET EXTENSEURS ENDOVASCULAIRES EXPANSIBLES FORMES AVEC CETTE MATIERE

(84) Designated Contracting States:
**AT BE CH DE DK ES FR GB GR IE IT LI LU MC NL PT SE**

(30) Priority: **27.06.1994 US 265794**

(43) Date of publication of application:
**16.04.1997 Bulletin 1997/16**

(73) Proprietor: **Bard Peripheral Vascular, Inc.**
**Tempe, AZ 85280 (US)**

(72) Inventor: **COLONE, William, M.**
**Phoenix, AZ 85048 (US)**

(74) Representative: **Deans, Michael John Percy et al**
**Lloyd Wise, Tregear & Co.,**
**Commonwealth House,**
**1-19 New Oxford Street**
**London WC1A 1LW (GB)**

(56) References cited:
| | |
|---|---|
| EP-A- 0 059 619 | EP-A- 0 269 449 |
| WO-A-94/04097 | US-A- 4 110 392 |
| US-A- 5 061 276 | US-A- 5 071 609 |
| US-A- 5 217 483 | US-A- 5 282 860 |
| US-A- 5 383 928 | |

EP 0 767 684 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

This invention relates to polytetrafluoroethylene (hereinafter PTFE) materials which, after being radially expanded, retain their the structural integrity. More particularly, the invention relates to extruded, stretched, sintered tubular PTFE materials suited for use in the medical field as liners and covers for expandable stents.

The use of expandable endovascular stents to open and support aortic blood vessels is well known in the art. Such stents, which are typically made from stainless steel, are thrombogenic and tend to occlude due to growth of tissue through the stent into the blood vessel. The length of such stents is also limited because of their rigidity. Consequently, liners and covers have been sought for use in conjunction with metallic stents in order to shield the stent and to extend the length of anatomy which can be treated with the stent. The development of acceptable stent liners or covers has been slow because the liners or covers preferably must (1) expand with the stent, (2) be non-thrombogenic, (3) be biocompatible, (4) be inert, (5) have a low profile with the ability to expand up to about four times its original dimension, (6) be expandable at low pressures of less than five to ten atmospheres to reduce the risk of injury to the patient, (7) retain its physical properties and structural strength after being expanded, (8) generally not alter its length after being expanded, (9) be impervious to blood at physiological pressures, (10) conform to host anatomy when expanded, (11) resist the growth of bodily tissue therethrough, (12) be able to carry radiopaque markings for location during fluoroscopy.

Paste-formed, extruded tubular PTFE products are well known, as are paste extrusion and paste forming manufacturing processes for producing such products. During such manufacturing processes, a PTFE resin is mixed with a liquid lubricant. A preformed resin/lubricant charge is then produced and extruded through an annular orifice to produce an unsintered PTFE tube. The extruded tube is heated to remove the lubricant and produce a porous, unsintered PTFE tube. The tube typically has a density of from 1.5 to about 1.75 gm/cc and accompanying porosity of 39% to 26%. If the unsintered tube is sintered by heating the tube to a temperature above its crystalline melting temperature, a nonporous tube results. See U.S. Patent Nos. 3,953,566, 3,962,153, 4,110,392, 4,187,390, 4,283,448, 4,385,093, 4,478,665, 4,482,516, 4,877,661, and 5,026,513.

EP-A-0269449 discloses an interpenetrated matrix of a PTFE material and an elastomer which is formed into a composite material.

US-A-5071609 also discloses an interpenetrated matrix of PTFE and elastomer which is formed into a composite material.

In the medical field, PTFE products are used as replacement veins and arteries. PTFE is inert, is non-thrombogenic, and has other characteristics desirable for a stent cover or liner. Commercially available PTFE medical tubular products have, however, significant radial strength and are not readily dilated. Conventional PTFE tubes typically have a high radial strength and rapidly lose their tensile strength and become weak and thin after being dilated by only small amounts.

Accordingly, it would be highly desirable to provide improved PTFE products which can be readily expanded and which, after being expanded, substantially retain their tensile strength and other physical properties which make the use of PTFE in the body desirable.

Summary of the Invention

I have discovered new PTFE products and a process and composition for producing the same. The new PTFE products can be significantly expanded to configurations which retain their structural integrity and which substantially retain their tensile strength and other desirable physical properties. As discussed in detail in the examples below, the new PTFE products have an unusually low REC (Radial Expansion Coefficient) and RER (Radial Expansion Ratio), permitting thin-walled PTFE tubes to expand about 50% to 400%, or more, before the tubes lose their structural integrity and suffer a rapid decline in tensile strength. The new PTFE products are pre-dilated to insure that an RER value equal to one is achieved.

There is disclosed herein a tube-form medical implant of porous material comprising crystalline polytetrafluoroethylene (PTFE) polymer, which material has a microstructure of nodes interconnected by fibrils in the form of a PTFE tube in a pre-dilated and contracted diameter state as a result of radial expansion of a longitudinally expanded, sintered PTFE tube, followed by contraction produced by re-sintering, the tube-form implant being expandable in use from the contracted diameter to a substantially larger implantation diameter by application of radial force.

In one aspect, the invention features a method for producing a radially expandable tube-form medical implant formed of extruded crystalline polytetrafluoroethylene polymer having a microstructure of modes interconnected by fibrils, comprising the steps of: (a) extruding a lubricant/polytetrafluoroethylene resin blend to form a tubing having a longitudinal axis, a primary inner diameter, and a primary length; (b) heating the tubing to remove the lubricant; (c) stretching the tubing along the longitudinal axis to produce an elongated tubing having a secondary length greater than the primary length;

(d) radially expanding the sintered tubing to produce radially expanded tubing having an expanded inner diameter that is at least two times the primary inner diameter; and (e) sintering the radially expanded tubing to contract the radially expanded tubing to a contract diameter substantially the same as the primary inner diameter, resulting in said implant exhibiting a radial expansion ratio (PER) of 1.0. Preferably the elongated tubing is sintered between steps (c) and (d).

In another aspect, the invention features a method for producing a porous tube consisting essentially of highly crystalline polytetrafluoroethylene polymer comprising the steps of: (a) extruding a lubricant/polytetrafluoro-ethylene resin blend to form a tubing having a longitudinal axis, a primary inner diameter, and a primary length; (b) heating the tubing to remove the lubricant; (c) stretching the tubing along the longitudinal axis to produce an elongated tubing having a secondary length greater than the primary length; (d) restraining the elongate tubing to prevent the elongate tubing from longitudinally contracting during sintering; (e) sintering the elongated tubing to produce a sintered tubing having a length substantially equivalent to the secondary length; (f) radially expanding the sintered tubing to produce radially expanded tubing have an expanded inner diameter greater than the primary inner diameter; and (g) sintering the radially expanded tubing to contract the radially expanded tubing and produce a tubing having an inner diameter substantially equivalent to the primary inner diameter, resulting in said implant exhibiting a radial expansion ratio of 1.0.

The sintered radially expanded tubing may be longitudinally stretched after step (g). The ends of the radially expanded tubing may be restrained, at a step between steps (f) and (g), to maintain the length of the radially expanded tubing when the radially expanded tubing is sintered.

In another aspect, the invention features a radially expandable tube-form medical implant obtainable by a method as described above.

In another aspect, the invention features an expandable tubular medical implant comprising porous material of crystalline polytetrafluoroethylene polymer having a microstructure of nodes interconnected by fibrils, the tubular implant being permanently, radially expandable from a small delivery diameter, which is of low profile suitable for delivery to an implantation site inside a blood vessel, to a more than 50% larger implantation diameter, which is suitable for implantation inside a blood vessel, by application of outward radial force, the tubular medical implant, after expansion to the implantation diameter, substantially retaining its expanded size, tensile strength, and structural integrity so that increase in the outward radial force is required before further radial expansion can occur wherein the expandable tubular medical implant exhibits a radial expansion ratio of 1.0, and wherein the radial expansion ratio is the radial expansion coefficient divided by the radial expansion limit, the radial expansion coefficient being the maximum inflation pressure of the tubular medical implant without loss of structural integrity divided by the percentage radial dilatation of the implant at the maximum, inflation pressure and the radial expansion limit being the pressure of the implant at which it bursts divided by the percentage radial dilatation of the implant at the burst pressure.

There is also disclosed herein a readily dilatable tube having a low profile delivery diameter, the tube constructed as a tubular implant for use in concentric relationship with an expandable stent, the tube, upon expansion by more than 50% to an implantation diameter, retaining structural integrity and substantial strength, the tube comprising a tube of expanded polytetrafluoroethylene (PTFE) in a pre-dilated and contracted diameter state as a result of radial expansion of a longitudinally expanded, sintered PTFE tube, followed by contraction produced by re-sintering.

In a further aspect, the invention features an expandable endovascular stent having a contracted diameter state with an outer diameter suitable for delivery through the vasculature of a patient to a desired site and an expanded diameter state with an outer diameter suitable for supporting a blood vessel at the desired site; the stent comprising: a radially expandable tube-form medical implant, in accordance with one of the inventive aspects described above, having an interior wall surface and an exterior wall surface; and at least one tubular, radially expandable support structure coupled to the tubular medical implant, the support structure having an interior wall surface and an exterior wall surface.

Embodiments may include one or more of the following features. The implant is preferably formed from an extruded tube having an original inner diameter, in which the pre-dilation increased the inner diameter to at least two to at least five times the original inner diameter. The tube-form implant preferably has a Radial Expansion Coefficient (REC) of less than 13.8 kPa/% (2.0 psi/%), preferably less than 11.8 kPa/% (1.7 psi/%), more preferably less than 10.4 kPa/% (1.5 psi/%), and still more preferably less than 6.9 kPa/% (1.0 psi/%). The tube-form implant preferably has a ratio of Reduction Ratio (RR) to Lubricant Level (LL) of less than 5. The tube-form implant preferably has the characteristic of maintaining its structural integrity until the tube-form implant is radially expanded by more than 50%, preferably by more than 75%, more preferably by more than 100%, and still more preferably by more than 150%. The tubular implant preferably has the characteristic of substantially retaining its expanded size, structural integrity, and substantial tensile strength when expanded 200% beyond its delivery diameter so that increase in outward radial force is required before further radial expansion can occur.

In some embodiments, the expandable tubular implant is preferably combined with an endovascular stent, e. g., in the form or a cover or a liner, or both, for an endovascular stent. The expandable tubular implant preferably has the characteristic of being expandable by an angioplasty balloon catheter at a pressure of about 500 kPa to 1520 kPa (5 to 15 atmospheres). The tubular implant preferably has the characteristic that its longitudinal length does not substantially change as a result of radial expansion. The medical implant preferably has the characteristic of maintaining its structural integrity when expanded by 50%-400%, or more, so that increase in the radial force is required before further expansion can occur.

In some preferred embodiments, the tubular medical implant is disposed on the exterior wall surface of the at least one support structure. The tubular medical implant has first and second ends, and, in some preferred embodiments, there are preferably two of the support structures disposed within the interior wall surface of the medical implant at first and second ends thereof, respectively. In some embodiments, the tubular medical implant is preferably disposed within the interior wall surface of the at least one support structure. In some embodiments, an extension of the tubular medical implant wraps around an end of the at least one support structure, from the interior wall surface to the exterior wall surface of the support structure. In some other embodiments, the first tubular medical implant is disposed within the interior wall surface of the at least one support structure and a second tubular implant is disposed about the exterior wall surface of the at least one support structure.

In some preferred embodiments, the at least one radially expandable support structure retains its shape upon expansion to the expanded diameter state as a result of deformation beyond the elastic limit of the material forming the support structure. In some embodiments, the radially expandable support structure is radially self-expandable to the expanded diameter state. In some preferred embodiments the radially expandable support structure is self-expanding as a result of thermal activation. The support structure is preferably formed from stainless steel or nitinol. The tubular medical implant may preferably be coated with a pharmacological agent.

Embodiments of the invention may include one or more of the following advantages. The invention provides improved PTFE products which are amenable to use as liners and covers for expandable stents. The invention also provides improved tubular PTFE products which substantially retain their structural integrity after the products are radially expanded. The invention enables extension of the length of anatomy which can be treated with an expandable stent.

The following examples are presented to illustrate the presently preferred embodiments of and practice of the invention and not by way of limitation of the scope of the invention; other features and advantages will become apparent from the following description and from the claims.

Brief Description of the Drawings

Fig. 1 is a somewhat diagrammatic view of a preform and an extruded tube.

Figs. 2-2A are diagrammatic side views of an endovascular stent formed with radially expanded pre-dilated polytetrafluoroethylene in a contracted diameter state and an expanded diameter state, respectively.

Figs. 3-3A are diagrammatic side views of an endovascular stent formed with radially expanded pre-dilated polytetrafluoroethylene in a contracted diameter state and an expanded diameter state, respectively.

Figs. 4-4A are diagrammatic side views of an endovascular stent formed with radially expanded pre-dilated polytetrafluoroethylene in a contracted diameter state and an expanded diameter state, respectively.

Figs. 5-5A are diagrammatic side views of an endovascular stent formed with radially expanded pre-dilated polytetrafluoroethylene in a contracted diameter state and an expanded diameter state, respectively.

Figs. 6-6A are diagrammatic side views of an endovascular stent formed with radially expanded pre-dilated polytetrafluoroethylene in a contracted diameter state and an expanded diameter state, respectively.

Figs. 7-7A are diagrammatic side views of an endovascular stent formed with radially expanded pre-dilated polytetrafluoroethylene in a contracted diameter state and an expanded diameter state, respectively.

Description of the Preferred Embodiments

Examples 1-17, below, concern expandable PTFE material formed as a result of stretching and subsequent sintering, as described in the inventor's co-pending application U.S. Serial No. 08/239,239, filed May 6, 1994. Examples 18-23, below, concern expandable PTFE material formed as a result of expansion in a first dimension, sintering, pre-dilating in a second dimension, and re-sintering.

Porous, Highly Crystalline PTFE:

EXAMPLE 1 (for comparative purposes only)

One hundred grams of FLUON CD123 resin produced by ICI Americas, Inc. was sifted through a No. 10 sieve (nominal wire diameter 0.9 mm) and then blended at room temperature with twenty-five grams of ISOPAR M solvent produced by Exxon Corporation to produce a preform blend. Other lubricants well known in the art includes VM&P NAPHTHA (boiling point (bp) 118-130°C), ISOPAR (Registered trademark of Exxon Corporation), ISOPAR 3 G (bp 159-174°C), ISOPAR H (bp 176-189°C), Low Odor Paraffin Solvent (bp 191-246°C), and SHELLSOL (Trademark of Shell Oil) K.

The resulting preform blend was allowed to sit for over eight hours before being re-sifted through a No. 10 sieve (nominal wire diameter 0.9 mm). The lubricant level (LL) equals the weight of solvent used divided by the weight of resin used, which means the lubricant level utilized in this Example 1 was 25%. In the practice of the invention the lubricant level is normally in the range of 16% to 35%, and is presently preferably in the range of about 18% to 25%.

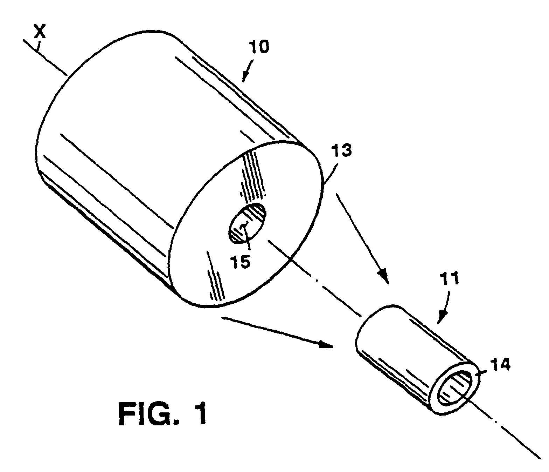

Referring to Fig. 1, a preform charge 10 was created by compacting the preform blend under 1.38 to 2.76 MPa (200 to 400 psi) for approximately one minute in a stainless steel cylinder containing a center shaft. The center shaft extended along the centerline X of and was concentric with the cylinder. The resulting preform charge 10 was a hollow cylindrical mass having a doughnut shaped circular cross sectional area 13, as shown in the drawing. The cylindrical hollow 15 in the preform was occupied by the center shaft. The preform charge was then loaded into a cylindrical barrel in a ram extruder and was extruded into several individual lengths of cylindrical thin-walled tubing 11 at a reduction ratio (RR) of 125:1. The total length of tubing 11 produced from the preform charge was about 6 metres (twenty feet). The extruded tubing had a microstructure characterized by nodes interconnected by fibrils. The reduction ratio equals the ratio of the cross sectional area 13 of the preform to the cross sectional area 14 of the wall of the tubing 11. In the practice of the invention, the RR is less than 200 or 300 to 1; preferably equal to or less than 125:1. The ratio of the RR to the LL in the practice of the invention is preferably less than five. In prior art preform blends the ratio of the RR to the LL is normally greater than five, and is typically nine or greater.

The solvent was removed from the extruded tubing by placing the tubes in a forced-air circulation electric oven at 255 degrees C for thirty minutes. As used herein, the length of the tube after it is extruded and heated at 255 degrees C to remove the solvent is termed the original length of the tube.

After being heated to 255 degrees C, each tube was heated to 290 degrees C for five minutes and then stretched longitudinally at rate of about 100% per second to a length four times the original length of the tube. Each tube can, if desired, be stretched at a rate in the range of 5% to 500% per second and stretched to a length in the range of two to six times the original length of the tube.

The stretched porous tubes were then sintered at approximately 300 degrees C for forty-five to 90 seconds. The sintering crystallized the PTFE and increased the strength of the porous tubes. During sintering each end of the tubes was restrained to prevent longitudinal shrinkage of the tube. The resulting stretched, sintered, porous tubes consisted essentially of highly crystalline PTFE polymer and had a microstructure characterized by nodes interconnected by fibrils.

The FLUON CD123 resin is a white free-flowing powder made by coagulation of an aqueous dispersion of polytetrafluoroethylene (PTFE). It is designed for paste extrusion with volatile hydrocarbon lubricants for applications in which opacity in the sintered article is not a problem. FLUON CD123 has a relatively high molecular weight. Unsintered extrudates exhibit good green strength.

| TYPICAL PROPERTIES OF FLUON CD 123 | | | |
|---|---|---|---|
| Property | Nominal Value | Units | Test Method |
| Apparent Density | 500 | grams/liter | ASTM D 1457-83 |
| Median Particle Size | 500 | microns | ASTM D 1457-83 |
| Melting Point | 327 | °C | ASTM D 1457-83 |
| Color | White | | |
| Specific Gravity | 2.16-2.18 | | ASTM D 1457-83 |
| Moisture Content (Max.) | 0.04 | % | ASTM D 1457-83 |
| Extrusion Pressure | $1.03 \times 10^6$ (15000) | Pa (psi) | |

EXAMPLE 2 (for comparative purposes only)

Example 1 was repeated except that twenty grams of ISOPAR M solvent was utilized instead of twenty-five grams and the pre-form charge was extruded at a reduction ratio (RR) of 91:1 into cylindrical thin-walled tubing. Approximately 6 metres (twenty feet) of cylindrical tubing was produced.

EXAMPLE 3 (for comparative purposes only)

Example 1 was repeated except that eighteen grams of ISOPAR M solvent was utilized instead of twenty-five grams and the pre-form charge was extruded at a reduction ratio (RR) of 48:1 into cylindrical thin-walled tubing. Approximately 3 metres (ten feet) of thin-walled tubing was produced.

EXAMPLE 4 (for comparative purposes only)

Example 1 was repeated except that eighteen grams of ISOPAR M solvent was utilized instead of twenty-five grams; ninety-five grams of CD123 was utilized instead of one hundred grams; five grams of CD509 was combined with the ISOPAR M solvent and the CD123; and, the resulting pre-form charges was extruded at a reduction ratio (RR) of 48:1 into cylindrical thin-walled tubing. Approximately 6 metres (ten feet) of thin-walled tubing was produced.
The FLUON CD509 resin is a white, free-flowing powder made by coagulation of an aqueous dispersion of polytetrafluoroethylene (PTFE). It is designed for paste extrusion at medium to high reduction ratios where high sintering rates are desirable.

| TYPICAL PROPERTIES OF FLUON CD 123 | | | |
|---|---|---|---|
| Property | Nominal Value | Units | Test Method |
| Apparent Density | 500 | grams/liter | ASTM D 1457-83 |
| Median Particle Size | 500 | microns | ASTM D 1457-83 |
| Melting Point | 327 | °C | ASTM D 1457-83 |
| Color | White | | |
| Specific Gravity | 2.18-2.20 | | ASTM D 1457-83 |
| Moisture Content (Max.) | 0.04 | % | ASTM D 1457-83 |
| Extrusion Pressure | 60.0 (8700) | Pa (psi) | |

EXAMPLE 5 (for comparative purposes only)

Three tubes approximately thirty-five centimeters long produced in Example 1 were each tested as follows.
An appropriate size angioplasty balloon catheter manufactured by Boston Scientific was placed in the inner lumen of the tube and was inflated with water with a standard MONARCH endoflater at a rate of approximately ten psi per second. Merit Medical manufacture the MONARCH endoflater. The balloon was about four centimeters long. As is well known, the balloon catheter is normally inserted in a blood vessel by first inserting a wire in a vessel; then inserting a vessel dilator along the wire into the vessel; removing the vessel dilator; inserting an introducer sleeve along the wire into the vessel; inserting the balloon; removing the introducer sleeve; inflating the balloon; deflating the balloon; removing the balloon; and removing the wire. A similar procedure was used while utilizing the balloon catheter to test the PTFE tubes of Example 1.
The balloon catheter did not apply an outward expansion force against the tube until the catheter was inflated under pressure with water. Inflation of the balloon (and the concomitant increase in inflation pressure) was stopped at predetermined pressure intervals of 101 kPa or 152 kPa (one or one-half atmosphere pressure) to measure the outside diameter of each tube. Each tube was dilated until it burst.
The actual inflation pressure was observed on a digital pressure gauge and recorded. The percent dilatation was calculated by measuring the tubing outside diameter with digital calipers at each pressure interval and then using the following formula:

$$\% \text{ Dilatation} = [(D_d - D_i)/(D_i)] \times 100$$

where $D_i$ = initial tube diameter at pressure equal to zero
$D_d$ = measured dilated tubing diameter
From the raw data, REC (Radial Expansion Coefficient), REL (Radial Expansion Limit), and RER (Radial Expansion Ratio) were calculated and recorded along with the calculated reduction ratio to lubricant level ratio (RR/LL), where:

$P_{max}$ = Maximum Inflation Pressure

$P_{burst}$ = Burst Inflation Pressure
%RD = Percent Radial Dilatation
REC = $(P_{max})$/(% RD at $P_{max}$)
REL = $(P_{burst})$ / (% RD at $P_{burst}$)
RER = (REC)/(REL)

As used herein, a tube retains its structural integrity after being radially expanded as long as the tube requires the application of an increased inflation pressure before the amount of radial expansion of the tube increases. If a tube continues to expand when the amount of inflation pressure decreases, then the tube has lost its structural integrity. When the Pmax of a tube is exceeded, the tube loses its structural integrity. However, the loss in structural integrity results in degradations of physical properties which are significantly less than those which occur in prior art PTFE tubes. For example, at a percent dilatation of about 300% in Table I below, the tube still retains about 70% to 75% of its pre-dilatation tensile strength. Also, in Table I below, Tube No. 1 loses its structural integrity at an inflation pressure greater than 659 kPa (6.5 atm) ($P_{max}$). In Tables II and III below, Tubes No. 2 and 3, respectively also lose their structural integrity at an inflation pressure greater than 659 kPa (6.5 atm) (Pmax).

The following results were obtained for the three Example 1 tubes which were tested:

<u>Tube No. 1</u>

Table I:

| Tube No. 1 Measurements | | | |
|---|---|---|---|
| <u>Measurement</u> | <u>Inflation (kPa) Pressure ((Atm))</u> | <u>Diameter (mm)</u> | <u>% Dilatation</u> |
| 1 | 0 | 2.75 | - |
| 2 | 101 (1) | 2.75 | 0 |
| 3 | 203 (2) | 2.75 | 0 |
| 4 | 304 (3) | 3.05 | 11 |
| 5 | 355 (3.5) | 3.13 | 14 |
| 6 | 405 (4) | 3.20 | 16 |
| 7 | 456 (4.5) | 3.34 | 21 |
| 8 | 507 (5) | 3.37 | 23 |
| 9 | 557 (5.5) | 3.92 | 43 |
| 10 | 608 (6) | 4.62 | 68 |
| 11 | 659 (6.5) ($P_{max}$) | 5.34 | 94 |
| 12 | 456 (4.5) ($P_{burst}$) | 12.12 | 341 |
| REC = 659 kPa/94% = 7.01 kPa/% REL = 456 kPa/341% = 1.34 kPa/% RER = (7.01)/(1.34) = 5.4 REC = (6.5 atm x 14.7 psi/atm)/94% = 1.02 psi/% REL = (4.5 atm x 14.7 psi/atm)/341% = 0.19 psi/% RER = (1.02)/(0.19) = 5.4 | | | |

Tube No. 2

Table II:

| Tube No. 2 Measurements | | | |
|---|---|---|---|
| Measurement | Inflation (kPa) Pressure ((Atm)) | Diameter (mm) | % Dilatation |
| 1 | 0 | 2.67 | - |
| 2 | 101 (1) | 2.67 | 0 |
| 3 | 203 (2) | 2.87 | 7 |
| 4 | 304 (3) | 3.02 | 13 |
| 5 | 355 (3.5) | 3.02 | 13 |
| 6 | 405 (4) | 3.17 | 19 |
| 7 | 456 (4.5) | 3.23 | 21 |
| 8 | 507 (5) | 3.40 | 27 |
| 9 | 557 (5.5) | 3.64 | 36 |
| 10 | 608 (6) | 4.77 | 79 |
| 11 | 659 (6.5) ($P_{max}$) | 5.51 | 106 |
| 12 | 456 (4.5) ($P_{burst}$) | 12.51 | 369 |
| REC = 659 kPa/106% = 6.22 kPa/%<br>REL = 456 kPa/369% = 1.24 kPa/%<br>RER = (6.22)/(1.24) = 5.0<br>REC = (6.5 atm x 14.7 psi/atm)/106% = 0.90 psi/%<br>REL = (4.5 atm x 14.7 psi/atm)/369% = 0.18 psi/%<br>PER = (0.90)/(0.18) = 5.0 | | | |

Tube No. 3

Table III:

| Tube No. 3 Measurements | | | |
|---|---|---|---|
| Measurement | Inflation (kPa) Pressure ((Atm)) | Diameter (mm) | % Dilatation |
| 1 | 0 | 2.75 | - |
| 2 | 101 (1) | 2.75 | 0 |
| 3 | 203 (2) | 2.75 | 0 |
| 4 | 304 (3) | 3.05 | 11 |
| 5 | 355 (3.5) | 3.13 | 14 |
| 6 | 405 (4) | 3.20 | 16 |
| 7 | 456 (4.5) | 3.34 | 21 |
| 8 | 507 (5) | 3.37 | 23 |
| 9 | 557 (5.5) | 3.92 | 43 |
| 10 | 608 (6) | 4.62 | 68 |

Table III:   (continued)

| Tube No. 3 Measurements | | | |
|---|---|---|---|
| Measurement | Inflation (kPa) Pressure ((Atm)) | Diameter (mm) | % Dilatation |
| 11 | 659 (6.5) ($P_{max}$) | 5.34 | 94 |
| 12 | 456 (4.5) ($P_{burst}$) | 12.97 | 372 |
| REC = 659 kPa/94% = 7.01 kPa/%<br>REL = 456 kPa/372% = 1.23 kPa/%<br>RER = (7.01)/(1.23) = 5.7<br>REC = (6.5 atm x 14.7 psi/atm)/94% = 0.90 psi/%<br>REL = (4.5 atm x 14.7 psi/atm)/371% = 0.18 psi/%<br>PER = (0.90)/(0.18) = 5.7 | | | |

EXAMPLE 6 (for comparative purposes only)

Three tubes approximately thirty-five centimeters long produced in Example 2 were each tested utilizing the equipment and procedure described in EXAMPLE 5. The following results were obtained for the three Example 2 tubes tested.

Tube No. 1

Table IV:

| Tube No. 1 Measurements | | | |
|---|---|---|---|
| Measurement | Inflation (kPa) Pressure ((Atm)) | Diameter (mm) | % Dilatation |
| 1 | 0 | 4.27 | - |
| 2 | 101 (1) | 4.27 | 0 |
| 3 | 203 (2) | 4.27 | 0 |
| 4 | 304 (3) | 4.35 | 2 |
| 5 | 355 (3.5) | 5.85 | 37 |
| 6 | 405 (4) ($P_{max}$) | 9.08 | 113 |
| 7 | 253 (2.5) ($P_{burst}$) | 16.39 | 284 |
| REC = 405 kPa/113% = 3.58 kPa/%<br>REL = 253 kPa/284% = 0.89 kPa/%<br>RER = (3.58)/(0.89) = 4.0<br>REC = (4.0 atm x 14.7 psi/atm)/113% = 0.52 psi/%<br>REL = (2.5 atm x 14.7 psi/atm)/284% = 0.13 psi/%<br>RER = (0.52)/(0.13) = 4.0 | | | |

Tube No. 2

Table V:

| Tube No. 2 Measurements | | | |
|---|---|---|---|
| Measurement | Inflation (kPa) Pressure ((Atm)) | Diameter (mm) | % Dilatation |
| 1 | 0 | 4.74 | - |
| 2 | 101 (1) | 4.74 | 0 |
| 3 | 203 (1) | 4.74 | 0 |
| 4 | 304 (3) | 5.49 | 16 |
| 5 | 355 (3.5) | 7.09 | 50 |
| 6 | 405 (4) ($P_{max}$) | 10.00 | 111 |
| 7 | 304 (3) ($P_{burst}$) | 20.52 | 333 |

REC = 405 kPa/111% = 3.65 kPa/%
REL = 304 kPa/333% = 0.91 kPa%
RER = (3.65)/(0.91) = 4.1
REC = (4 atm x 14.7 psi/atm)/111% = 0.53 psi/%
REL = (3 atm x 14.7 psi/atm)/333% = 0.13 psi/%
RER = (0.53)/(0.13) = 4.1

Tube No. 3

Table VI:

| Tube No. 3 Measurements | | | |
|---|---|---|---|
| Measurement | Inflation (kPa) Pressure ((Atm)) | Diameter (mm) | % Dilatation |
| 1 | 0 | 4.83 | - |
| 2 | 101 (1) | 4.83 | 0 |
| 3 | 203 (2) | 4.83 | 0 |
| 4 | 304 (3) | 5.23 | 8 |
| 5 | 355 (3.5) | 6.00 | 24 |
| 6 | 405 (4) ($P_{max}$) | 9.66 | 100 |
| 7 | 304 (3) ($P_{burst}$) | 18.12 | 275 |

REC = 405 kPa/100% = 4.05 kPa/%
REL = 304 kPa/275% = 1.11 kPa%
RER = (4.05)/(1.11) = 3.7
REC = (4 atm x 14.7 psi/atm)/100% = 0.59 psi/%
REL = (3 atm x 14.7 psi/atm/275% = 0.16 psi/%
RER = (0.59)/(0.16) = 3.7

EXAMPLE 7 (for comparative purposes only)

Two tubes approximately thirty-five centimeters long produced in Example 3 were each tested utilizing the equipment and procedure described in EXAMPLE 5. The following results were obtained for the two tubes tested.

Tube No. 1

Table VII:

| Tube No. 1 Measurements | | | |
|---|---|---|---|
| Measurement | Inflation (kPa) Pressure ((Atm)) | Diameter (mm) | % Dilation |
| 1 | 0 | 6.04 | - |
| 2 | 101 (1) | 6.28 | 4 |
| 3 | 152 (1.5) | 6.45 | 7 |
| 4 | 203 (2) | 6.79 | 12 |
| 5 | 253 (2.5) | 7.15 | 18 |
| 6 | 304 (3) | 7.39 | 22 |
| 7 | 355 (3.5) | 8.33 | 38 |
| 8 | 405 (4) ($P_{max}$) | 9.82 | 63 |
| 9 | 375 (3.7) ($P_{burst}$) | 24.77 | 310 |

REC = 405 kPa/63% = 6.43 kPa/%
REL = 375 kPa/310% = 1.21 kPa %
RER = (6.43)/(1.21) = 5.2
REC = (4.0 atm x 14.7 psi/atm)/63% = 0.93 psi/%
REL = (3.7 atm x 14.7 psi/atm)/310% - 0.18 psi/%
RER = (0.93)/0.18) = 5.2

Tube No. 2

Table VIII:

| Tube No. 2 Measurements | | | |
|---|---|---|---|
| Measurement | Inflation (kPa) Pressure ((Atm)) | Diameter (mm) | % Dilation |
| 1 | 0 | 5.99 | - |
| 2 | 101 (1) | 6.65 | 11 |
| 3 | 152 (1.5) | 6.76 | 13 |
| 4 | 203 (2) | 7.01 | 17 |
| 5 | 253 (2.5) | 7.31 | 22 |
| 6 | 304 (3) | 7.73 | 29 |
| 7 | 355 (3.5) | 8.43 | 41 |
| 8 | 405 (4) | 9.09 | 52 |
| 9 | 456 (4.5) ($P_{max}$) | 11. 17 | 89 |

Table VIII: (continued)

| Tube No. 2 Measurements | | | |
|---|---|---|---|
| Measurement | Inflation (kPa) Pressure ((Atm)) | Diameter (mm) | % Dilation |
| 10 | 395 (3.9) ($P_{burst}$) | 25.62 | 328 |
| REC = 456 kPa/89% = 5.12 kPa/% | | | |
| REL = 95 kPa/328% = 1.20 kPa/% | | | |
| RER = (5.12)/(1.20) = 4.5 | | | |
| REC = (4.5 atm x 14.7 psi/atm)/86% = 0.77 psi/% | | | |
| REL = (3.9 atm x 14.7 psi/atm)/328% = 0.17 psi/% | | | |
| RER = (0.77)/0.17) = 4.5 | | | |

EXAMPLE 8 (for comparative purposes only)

Two tubes approximately thirty-five centimeters long produced in Example 4 were each tested utilizing the equipment and procedure described in EXAMPLE 5. The following results were obtained for the two tubes tested.

Tube No. 1

Table IX:

| Tube No. 1 Measurements | | | |
|---|---|---|---|
| Measurement | Inflation (kPa) Pressure ((Atm)) | Diameter (mm) | % Dilation |
| 1 | 0 | 5.94 | - |
| 2 | 101 (1) | 6.40 | 8 |
| 3 | 152 (1.5) | 6.55 | 10 |
| 4 | 203 (2) | 7.02 | 18 |
| 5 | 253 (2.5) | 7.58 | 28 |
| 6 | 304 (3) | 9.51 | 60 |
| 7 | 355 (3.5) ($P_{max}$) | 13.15 | 121 |
| 8 | 294 (2.9) ($P_{burst}$) | 24.15 | 307 |
| REC = 355 kPa/121% = 2.93 kPa/% | | | |
| REL = 294 kPa/307% = 0.96 kPa/% | | | |
| RER = (2.93)/(0.96) = 3.1 | | | |
| REC = (3.5 atm x 14.7 psi/atm)/121% = 0.43 psi/% | | | |
| REL = (2.9 atm x 14.7 psi/atm)/307% = 0.14 psi/% | | | |
| RER = (0.43)/.014) = 3.1 | | | |

Tube No. 2

Table X:

| Tube No. 2 Measurements | | | |
|---|---|---|---|
| Measurement | Inflation (kPa) Pressure ((Atm)) | Diameter (mm) | % Dilation |
| 1 | 0 | 5.90 | - |

Table X: (continued)

| Tube No. 2 Measurements | | | |
|---|---|---|---|
| Measurement | Inflation (kPa) Pressure ((Atm)) | Diameter (mm) | % Dilation |
| 2 | 101 (1) | 6.41 | 9 |
| 3 | 152 (1.5) | 6.89 | 17 |
| 4 | 203 (2) | 7.09 | 20 |
| 5 | 253 (2.5) | 7.83 | 33 |
| 6 | 304 (3) | 8.34 | 41 |
| 7 | 355 (3.5) | 9.90 | 68 |
| 8 | 405 (4) ($P_{max}$) | 13.05 | 121 |
| 9 | 314 (3.1) ($P_{burst}$) | 24.76 | 320 |

REC = 405 kPa/121% = 3.34 kPa/%

REL = 314 kPa/320% = 0.98 kPa/%

RER = (3.34)/(0.98) = 3.5

REC = (4.0 atm x 14.7 psi/atm)/121% = 0.49 psi/%

REL = (3.1 atm x 14.7 psi/atm)/320% = 0.14 psi/%

RER = (0.49)/0.14 = 3.5

EXAMPLE 9 (for comparative purposes only)

Example 5 is repeated, except that after measurements are made at each pressure interval which causes the tube to dilate, the pressure is reduced by about one atmosphere to give the tube an opportunity to contract and five minutes later the diameter of the tube is remeasured. For example, after measurement no. 4 in Table I, the pressure is reduced to 203 kPa (two atmospheres) and five minutes later the diameter of the tube is remeasured; after example 5 in Table I, the pressure is reduced to 253 kPa (two and a half atmospheres) and five minutes later the diameter of the tube is remeasured; etc. Each time the diameter of the tube is remeasured, the diameter of the tube is reduced by about 10% or less from the measurement made when the pressure was 101 kPa (one atmosphere) greater. For example, after measurement no. 4 (3.05 mm) is taken in Table I, the water pressure is reduced to two atmospheres, and the diameter of the tube is measured five minutes later, the diameter of the tube is 2.75 mm.

EXAMPLE 10 (for comparative purposes only)

Example 1 is repeated except that the stretch rate is 10% per second instead of 100% per second.

EXAMPLE 11 (for comparative purposes only)

Example 1 is repeated except that the stretch rate is 300% per second instead of 100% per second.

EXAMPLE 12 (for comparative purposes only)

Example 1 is repeated except that the tube is stretched to three times its original length instead of four times its original length.

EXAMPLE 13 (for comparative purposes only)

Example 1 is repeated except that the tube is stretched to six times its original length instead of four times its original length.

EXAMPLE 14 (for comparative purposes only)

Example 5 is repeated utilizing tubes produced during Example 10. Similar results are obtained.

EXAMPLE 15 (for comparative purposes only)

Example 5 is repeated utilizing tubes produced during Example 11. Similar results are obtained.

EXAMPLE 16 (for comparative purposes only)

Example 5 is repeated utilizing tubes produced during Example 12. Similar results are obtained.

EXAMPLE 17 (for comparative purposes only)

Example 5 is repeated utilizing tubes produced during Example 13. Similar results are obtained.

Radially Pre-Dilated PTFE:

As described in the above examples, a porous hollow tube (or sheet or other shape) which consists essentially of highly crystalline polytetrafluoroethylene polymer and which has a microstructure characterized by nodes interconnected by fibrils is formed by extruding a lubricant/polytetra-fluoroethylene resin composition into a hollow tube, by heating the tube to remove the lubricant, by stretching the tube, and, while holding the tube in its stretched configuration, by sintering the tube at or above the melting temperature of the polytetrafluoroethylene resin to form a porous highly crystalline polytetrafluoroethylene polymer tube. This sintered porous tube of highly crystalline PTFE polymer has original inner and outer diameters.

As used herein, the terminology "radially pre-dilated" means that a porous highly crystalline polytetrafluoroethylene polymer tube is first radially dilated to increase the original inner (and outer) diameter of the tube and is then sintered to cause the dilated tube to contract radially to a configuration in which the diameter of the tube is less than the radially dilated diameter. When the radially dilated tube is sintered, the dilated tube is presently preferably contracted to a configuration in which the diameter of the tube substantially equals its original inner diameter. For example, and not by way of limitation, a sintered porous (the word "porous" indicates the extruded tube has been stretched along its longitudinal axis subsequent to being heated to remove lubricant and prior to being sintered to form highly crystalline PTFE polymer) tube of highly crystalline PTFE polymer having an inner diameter of six mm is radially pre-dilated by radially dilating the tube to an inner diameter (ID) of fifteen millimeters and by then sintering the tube to cause the tube to radially contract to an inner diameter of six mm. Similarly, the three mm ID sintered porous tube of highly crystalline PTFE polymer is radially pre-dilated by radially dilating the tube to an ID of six millimeters and by then sintering the tube to cause the tube to radially contract to an inner diameter to equal to its original inside diameter of three mm.

EXAMPLE 18

One hundred grams of FLUON CD 123 resin produced by ICI Americas, Inc, was sifted through a No. 10 sieve (nominal wire diameter 0.9 mm) and then blended at room temperature with twenty-five grams of ISOPAR M solvent (lubricant level 25%) produced by Exxon Corporation to produce a preform blend.

The resulting preform blend was allowed to sit for over eight hours before being re-sifted through a No. 10 sieve (nominal wire diameter 0.9 mm).

Referring again to Fig. 1, a preform charge 10 was created by compacting the preform blend under 1.38 to 2.76 MPa (200 to 400 psi) for approximately one minute in a stainless steel cylinder containing a center shaft. The center shaft extended along the centerline X of and was concentric with the cylinder. The resulting preform charge 10 was a hollow cylindrical mass having a doughnut-shaped circular cross sectional area 13, as shown in Fig. 1. The cylindrical hollow 15 in the preform was occupied by the center shaft. The preform charge was then loaded into a cylindrical barrel in a ran extruder and was extruded into several individual lengths of cylindrical thin-walled tubing 11 at a reduction ratio (RR) of 48:1. The total length of tubing 11 produced from the preform charge was about 6 metres (twenty feet). The extruded tubing had an inner diameter of five mm and had a microstructure characterized by nodes interconnected by fibrils.

The solvent was removed from the extruded tubing by placing the tubes in a forced-air circulation electric oven at 255 degrees C for thirty minutes. As used herein, the length of the tube after it is extruded and heated at 255 degrees C to remove the solvent is termed the original length of the tube.

A tube nine centimeters long was selected. After being heated to 255 degrees C, each tube was heated to 290 degrees C for five minutes and was then stretched longitudinally for the first time at rate of about 100% per second to a length four time the original length of the tube, i.e., to a tube thirty-six cm long.

The stretched porous thirty-six cm long tube was then sintered at approximately 360 degrees C for forty-five to ninety seconds. The sintering crystallized the PTFE and increased the strength of the porous tubes. During sintering each end of the tube was restrained to prevent longitudinal shrinkage of the tube. The resulting stretched, sintered, porous tubes consisted essentially of highly crystalline PTFE polymer and had a microstructure characterized by nodes interconnected by fibrils.

The sintered thirty-six cm long tube was pre-dilated by radially dilating the tube along its entire length to a twenty-four mm inner diameter. The tube was dilated by using an angioplasty balloon catheter in the manner described in Example 3, above.

The dilated tube was re-sintered at 355 degrees C for four minutes to cause the tube to contract radially and return to its original diameter of five mm and original length of nine cm. The tube was not restrained during sintering. Sintering the dilated tube at 355 degrees C completed the pre-dilation procedure.

The pre-dilated five mm diameter and nine cm long tube was heated to 300 degrees C for five minutes and stretched a second time to a length of eighteen centimeters.

EXAMPLE 19

The eighteen centimeter long tube produced in Example 18 was tested using the angioplasty balloon catheter and procedure described in Example 5. The results obtained are shown below in Table XI.

Table XI:

| Measurements for Example 18 Tube | | | |
|---|---|---|---|
| Measurement | Inflation (kPa) Pressure ((Atm)) | Diameter (mm) | % Dilation |
| 1 | 0 | 6.01 | - |
| 2 | 101 (1) | 6.01 | 0 |
| 3 | 203 (2) | 6.19 | 3 |
| 4 | 304 (3) | 6.80 | 13 |
| 5 | 405 (4) | 6.90 | 15 |
| 6 | 507 (5) | 7.01 | 17 |
| 7 | 608 (6) | 7.68 | 28 |
| 8 | 709 (7) | 8.40 | 40 |
| 9 | *760 (7.5) | 23.67 | 294 |
| REC = 760 kPa/294% = 2.59 kPa/%<br>REL = 760 kPa/294% = 2.59 kPa/%<br>RER = (2.59)/(2.59) = 1.0<br>REC = (7.5 atm x 14.7 psi/atm)/294% = 0.375 psi/%<br>REL = (7.5 atm x 14.7 psi/atm)/294% = 0.375 psi/%<br>RER = (0.375)/(0.375) = 1.0 | | | |

*$P_{max}$ and $P_{burst}$

From an endovascular grafting perspective, an RER of 1.0 is an ideal value because the maximum pressure is equal to the burst pressure. This permits the ready dilation of a polytetrafluoroethylene tube without exceeding the maximum pressure. Exceeding the maximum pressure results in the loss of structural integrity.

As would be appreciated by those of skill in the art, the amount by which an extruded tube is lengthened before it is sintered to produce highly crystalline polytetrafluoroethylene polymer can be varied as desired to control the amount of pressure required to dilate a tube. The more a tube is lengthened, the less dense the wall of the tube and the less pressure required to dilate the tube. Similarly, the amount by which the tube is pre-dilated can be varied as desired. In Example 18, the tube was pre-dilated to a diameter equal to about five times the original diameter and was lengthened the second time to a length (equal to twice the original extruded length) which was only one-half of the length (equal to four time the original extruded length) at which the tube was pre-dilated to 24 mm. As a result, the maximum inflation pressure occurred when the tube was radially dilated to an outer diameter of about 23.67 mm. The amount by which the tube is lengthened the second time and the amount of pre-dilated can be varied to alter the amount of tube dilation required to reach the maximum inflation pressure, Pmax.

EXAMPLE 20

Example 18 was repeated, except that after the pre-dilated tube was five mm inner diameter and nine cm long tube was heated to 300 degrees C for five minutes and stretched a second time, the tube was stretched to a length of thirteen and a half centimeters instead of a length of eighteen centimeters.

EXAMPLE 21

The thirteen and a half centimeters long tube produced in Example 20 was tested using an angioplasty balloon catheter and procedure described in Example 5. The results obtained are shown below in Table XII. The tubes are generally expandable by an angioplasty balloon catheter inflated to pressures of at least about 5 to 1520 kPa (5 to 15 atmospheres).

Table XII:

| Measurements for Example 20 Tube | | | |
|---|---|---|---|
| Measurement | Inflation (kPa) Pressure ((Atm)) | Diameter (mm) | % Dilation |
| 1 | 0 | 6.48 | - |
| 2 | 304 (3) | 6.48 | 0 |
| 3 | 405 (4) | 6.71 | 4 |
| 4 | 507 (5) | 6.71 | 4 |
| 5 | 608 (6) | 6.87 | 6 |
| 6 | 709 (7) | 6.87 | 6 |
| 7 | 811 (8) | 6.87 | 6 |
| 8 | 912 (9) | 7.17 | 11 |
| 9 | 1013 (10) | 7.83 | 21 |
| 10 | 1115 (11) | 8.68 | 34 |
| 11 | 1216 (12) | 11.92 | 84 |
| 12 | *1267 (12.5) | 24.28 | 275 |
| REC = 1267 kPa/275% = 4.61 kPa/% <br> REL = 1267kPa/275% = 4.61 kPa/% <br> RER = (4.61)/(4.61) = 1.0 <br> REC = (12.5 atm x 14.7 psi/atm)/275% = 0.69 psi/% <br> REL = (12.5 atm x 14.7 psi/atm)/275% = 0.69 psi/% <br> RER = (0.69)/(0.69) = 1.0 | | | |

\*$P_{max}$ and $P_{burst}$

The results in Table XII demonstrate how increasing the density of the wall of the tubing also increased the pressure needed to dilate the tubing. The density of the wall of the tube is increased by reducing the amount by which the tube is stretched. The Pmax and percent dilatation at Pmax can be adjusted to reasonable desired values by varying the amount by which the tube is pre-dilated and the amount by which the tube is stretched. The values of Pmax and percent dilatation at Pmax are limited by the physical properties of the polytetrafluoroethylene utilized to prepare the tubing.

EXAMPLE 22 (for comparative purposes only)

One hundred grams of FLUON CD123 resin produced by ICI Americas, Inc. was sifted through a No. 10 sieve (nominal wire diameter 0.9 mm) and then blended at room temperature with eighteen grams of ISOPAR M solvent (18% lubricant level) produced by Exxon Corporation to produce a preform blend.

The resulting preform blend was allowed to sit for over eight hours before being re-sifted through a No. 10 sieve (nominal wire diameter 0.9 mm). A preform charge 10 was created by compacting the preform blend under 1.38 to 2.76 MPa (200 to 400 psi) for approximately one minute in a stainless steel cylinder containing a center shaft. The center shaft extended along the centerline X of and was concentric with the cylinder. The resulting preform charge 10 (Fig. 1) was a hollow cylindrical mass having a doughnut-shaped circular cross sectional area 13, as shown in Fig. 1. The cylindrical hollow 15 in the preform was occupied by the center shaft. The preform charge was then loaded into a cylindrical barrel in a ram extruder and was extruded into several individual lengths of cylindrical thin-walled tubing 11 at a reduction ratio (RR) of 51:1. The total length of tubing 11 produced from the preform charge was about 6 metres (twenty feet). The extruded tubing had an inner diameter of three mm and had a microstructure characterized by nodes interconnected by fibrils.

The solvent was removed from the extruded tubing by placing the tubes in a force-air circulation electric oven at 255 degrees C for thirty minutes. As used herein, the length of the tube after it is extruded and heated at 255 degrees C to remove the solvent is termed the original length of the tube.

First and second tubes each nine centimeters long were selected. After being heated to 255 degrees C, each tube was heated to 290 degrees C for five minutes and was then stretched longitudinally for the first time at rate of about 100% per second to a length three times the original length of the tube, i.e., to a tube twenty-seven cm long.

The pair of stretched porous twenty-seven cm long tubes were then sintered at approximately 360 degrees C for forty-five to ninety seconds. The sintering crystallized the PTFE and increased the strength of the porous tubes. During sintering each end of the tube was restrained to prevent longitudinal shrinkage of the tube. The resulting stretched, sintered, porous tubes consisted essentially of highly crystalline PTFE polymer and had a microstructure characterized by nodes interconnected by fibrils.

The first sintered twenty-seven cm long tube was tested using the angioplasty balloon catheter and procedure described in Example 5. The results obtained are shown below in Table XIII.

Table XIII:

| Measurements for First Example 22 Tube | | | |
|---|---|---|---|
| Measurement | Inflation (kPa) Pressure ((Atm)) | Diameter (mm) | % Dilation |
| 1 | 0 | 5.34 | - |
| 2 | 101 (1) | 5.43 | 2 |
| 3 | 203 (2) | 5.43 | 2 |
| 4 | 304 (3) | 5.43 | 2 |
| 5 | 405 (4) | 5.43 | 2 |
| 6 | 507 (5) | 5.58 | 4 |
| 7 | 608 (6) ($P_{max}$) | 6.55 | 23 |
| 8 | 507 (5) ($P_{burst}$) | 20.76 | 291 |
| REC = 26.4 kPa/% (3.83 psi/%) | | | |
| REL = 1.72 kPa/% (0.25 psi/%) | | | |
| RER = 15.3 | | | |

The second sintered twenty-seven cm long tube was pre-dilated by radially dilating the tube along its entire length to a ten mm inner diameter. The tube was dilated by using an angioplasty balloon catheter in the manner described in Example 3.

The dilated tube was re-sintered at 355 degrees C for four minutes to cause the tube to radially contract and return to its original diameter of three mm and original length of nine cm. Sintering the dilated tube at 355 degrees C completed the pre-dilating procedure.

The pre-dilated five mm diameter and nine cm long tube was heated to 300 degrees C for five minutes and stretched a second time to a length of eighteen centimeters. The eighteen centimeter pre-dilated tube produced in this Example 22 was then tested using the angioplasty balloon catheter and procedure described in Example 5. The results obtained are.shown below in Table XIV.

Table XIV:

| Measurements for Second Example 22 Tube (Pre-Dilated) | | | |
|---|---|---|---|
| Measurement | Inflation (kPa) Pressure ((Atm)) | Diameter (mm) | % Dilation |
| 1 | 0 | 5.38 | - |
| 2 | 101 (1) | 5.38 | 0 |
| 3 | 203 (2) | 5.38 | 0 |
| 4 | 304 (3) | 5.38 | 0 |
| 5 | 405 (4) | 5.38 | 0 |
| 6 | 507 (5) | 5.38 | 0 |
| 7 | 608 (6) ($P_{max}$) | 6.56 | 22 |
| 8 | 557 (5.5) ($P_{burst}$) | 19.26 | 258 |
| REC = 27.6 kPa/% (4.01 psi/%) REL = 2.14 kPa/% (0.31 psi/%) RER = 12.9 | | | |

## EXAMPLE 23

One hundred grams of FLUON CD123 resin produced by ICI Americas, Inc. was sifted through a No. 10 sieve (nominal wire diameter 0.9 mm) and then blended at room temperature with eighteen grams of ISOPAR M solvent (18% lubricant level) produced by Exxon Corporation to produce a preform blend.

The resulting preform blend was allowed to sit for over eight hours before being re-sifted through a No. 10 sieve (nominal wire diameter 0.9 mm). A preform charge 10 (Fig. 1) was created by compacting the preform blend under 1.38 to 2.76 MPa (200 to 400 psi) for approximately one minute in a stainless steel cylinder containing a center shaft. The center shaft extended along the centerline X of and was concentric with the cylinder. The resulting preform charge 10 was a hollow cylindrical mass having a doughnut-shaped circular cross sectional area 13, as shown in Fig. 1. The cylindrical hollow 15 in the preform was occupied by the center shaft. The preform charge was then loaded into a cylindrical barrel in a ram extruder and was extruded into several individual lengths of cylindrical thin-walled tubing 11 at a reduction ratio (RR) of 51:1. The total length of tubing 11 produced from the preform charge was about 6 metres (twenty feet). The extruded tubing had an inner diameter of three mm and had a microstructure characterized by nodes interconnected by fibrils.

The solvent was removed from the extruded tubing by placing the tubes in a forced-air circulation electric oven at 255 degrees C for thirty minutes. As used herein, the length of the tube after it is extruded and heated at 255 degrees C to remove the solvent is termed the original length of the tube.

First, second, and third tubes each nine centimeters long were selected. After being heated to 255 degrees C, each tube was heated to 290 degrees C for five minutes and was then stretched longitudinally for the first time at a rate of about 100% per second to a length seven times the original length of the tube, i.e., to a tube sixty-three cm long.

The trio of stretched porous sixty-three cm long tubes were then sintered at approximately 360 degrees C for forty-five to ninety seconds. The sintering crystallized the PTFE and increased the strength of the porous tubes. During sintering each end of the tube was restrained to prevent longitudinal shrinkage of the tube. The resulting stretched, sintered, porous tubes consisted essentially of highly crystalline PTFE polymer and had a microstructure characterized by nodes interconnected by fibrils.

The first sintered sixty-three cm long tube was tested using the angioplasty balloon catheter and procedure described in Example 5. The angioplasty balloon was about four cm long. The results obtained are shown below in Table XV.

Table XV:

| Measurements for First Example 23 Tube (Tube Not Pre-Dilated) | | | |
|---|---|---|---|
| Measurement | Inflation (kPa) Pressure ((Atm)) | Diameter (mm) | % Dilation |
| 1 | 0 | 5.66 | - |
| 2 | 101 (1) | 5.66 | 0 |
| 3 | 203 (2) | 5.66 | 0 |
| 4 | 304 (3) ($P_{max}$) | 5.76 | 19 |
| 5 | 284 (2.8) ($P_{burst}$) | 19.92 | 252 |
| REC = 16.0 kPa/% (2.32 psi/%) REL = 1.10 kPa/% (0.16 psi/%) RER = 14.5 | | | |

The second sintered sixty-three cm long tube was pre-dilated by radially dilating the tube along its entire length to a ten mm inner diameter. The tube was dilated by using an angioplasty balloon catheter in the manner described in Example 3.

The dilated second tube was restrained longitudinally (so the tube could not contract to a shorter length) and was re-sintered at 350 degrees C for thirty seconds to cause the second tube to return to its original diameter of three mm. After sintering at 350 degrees C, the length of the second tube was the same, i.e., sixty-three cm. Sintering the dilated second tube at 350 degrees C for thirty seconds completed the pre-dilation procedure for the second tube.

The pre-dilated three mm diameter and sixty-three cm long second tube was tested using the angioplasty balloon catheter and procedure described in Example 5. The angioplasty balloon was four cm long. The results obtained are shown below in Table XVI.

Table XVI:

| Measurements for Second Example 23 Tube (Pre-Dilated; Sintered Under Longitudinal Restraint) | | | |
|---|---|---|---|
| Measurement | Inflation (kPa) Pressure ((Atm)) | Diameter (mm) | % Dilation |
| 1 | 0 | 5.37 | - |
| 2 | 101 (1) | 5.72 | 7 |
| 3 | 203 (2) | 5.82 | 8 |
| 4 | 304 (3) ($P_{max}$) | 7.78 | 45 |
| 5 | 284 (2.8) ($P_{burst}$) | 20.78 | 287 |
| REC = 6.76 kPa/% (0.98 psi/%) REL = 0.97 kPa/% (0.14 psi/%) PER = 7.0 | | | |

The third sintered sixty-three cm long tube was pre-dilated by radially dilating the tube along its entire length to a fifteen mm inner diameter. The tube was dilated by using an angioplasty balloon catheter in the manner described in Example 3.

The dilated third tube was restrained longitudinally (so the tube could not contract to a shorter length) and was re-sintered at 350 degrees C for thirty seconds to cause the tube to return to its original diameter of three mm. The length of the third tube remained the same, i.e., sixty-three cm. Sintering the dilated third tube at 350 degrees C for thirty seconds completed the pre-dilation procedure for the third tube.

The pre-dilated three mm diameter and sixty-three cm long third tube was tested using the angioplasty balloon catheter and procedure described in Example 5. The angioplasty balloon was four cm long. The results obtained are shown below in Table XVII.

# EP 0 767 684 B1

Table XVII:

| Measurements for Third Example 23 Tube (Pre-Dilated; Sintered Under Longitudinal Restraint) | | | |
|---|---|---|---|
| Measurement | Inflation (kPa) Pressure ((Atm)) | Diameter (mm) | % Dilation |
| 1 | 0 | 6.00 | - |
| 2 | 101 (1) | 6.00 | 0 |
| 3 | 203 (2) | 7.68 | 28 |
| 4 | *253 (2.5) | 21.14 | 252 |
| REC = 1.03 kPa/% (0.15 psi/%) REL = 1.03 kPa/% (0.15 psi/%) PER = 1.0 | | | |

*$P_{max}$ and $P_{burst}$

As evidenced by Example 23, the PTFE tubing had to be significantly pre-dilated, i.e., up to a diameter equal to five times the original diameter, in order to obtain an RER of 1. The amount of pre-dilation necessary to obtain an RER of 1 can be determined for each particular lubricant/PTFE resin composition and for each selected longitudinal expansion (stretching) of an extruded tube which is used prior to sintering of an extruded tube to produce a high crystalline PTFE structure. A tube having an RER of 1 will not rupture because when the tube is expanded to a diameter less than the diameter to which the tube is expanded during pre-dilation, the tube retains its structural integrity.

As would be appreciated by those of skill in the art, after a PTFE tube (or sheet or other shaped PTFE article) is (a) lengthened to a first length by being stretched along its longitudinal axis prior to being initially sintered to for a highly crystalline PTFE polymer, and (b) pre-dilated, the tube can again be lengthened by being stretched a second time along it longitudinal axis to a second length less than, equal to, or greater than the first length.

Expandable Endovascular Stents:

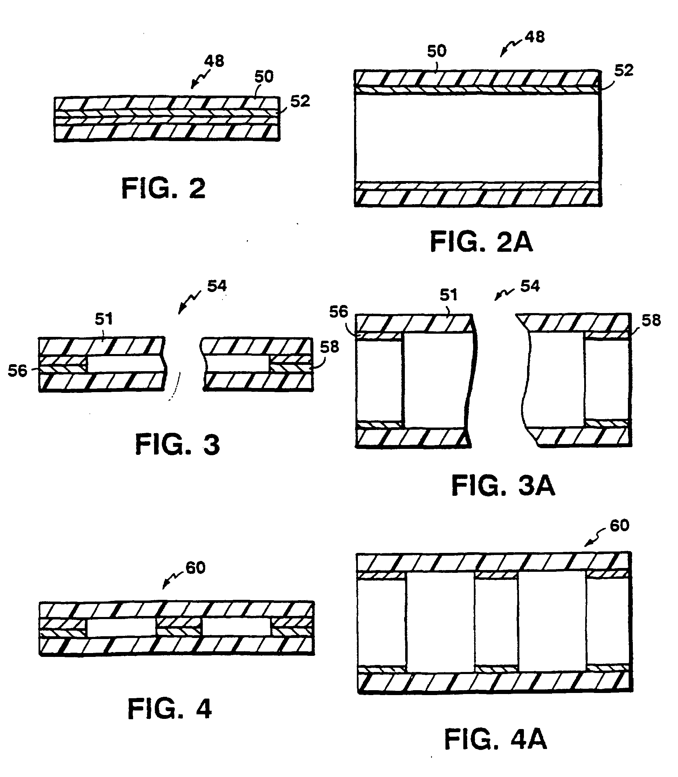

Referring to Figs. 2-7A, radially expandable PTFE tubes formed in accordance with the above examples are amenable for use with an endovascular stent as a liner or a cover, or both. Figs. 2-7A are merely intended to be diagrammatic and not limiting; in practical embodiments, there would be a lumen defined through the stents shown in Figs. 2, 3, 4, 5, 6, and 7 for receiving, e.g., a guidewire or a balloon of an angioplasty catheter, which would be used to deliver the stent to the implantation site. Such stents may be used to expand a partially occluded segment of a blood vessel, or other body passageway; it may also be used for other purposes, such as: a supportive graft placed within blocked arteries opened by recanalization; a support within opened vessels which were occluded by inoperative cancers; a support within veins treated for portal hypertension; a supportive graft for the esophagus, intestine, ureters, urethra, and the bile duct; and as a graft for sealing, e.g., an arterio-venous fistula. Such stents are characterized by a contracted diameter state, which is suitable for delivery through the vasculature of a patient to a desired site, and an expanded diameter state, which is suitable for support of a blood vessel at the desired site. The length and expanded diameter of such stents will depend on the anatomy of the desired implantation site. In some preferred embodiments, such stents have a length of about 0.5-45 cm, a contracted diameter of about 1-6 mm, and an expanded diameter of about 2.5-30 mm. The endovascular stents are preferably delivered to the desired implantation site through an introducer sheath having an outer diameter of about 5-20 Fr.

As shown in Figs. 2 and 2A, a stent 48 includes a radially pre-dilated PTFE tube 50 disposed as a cover about an endovascular stent support structure 52. Stent 48 is useful for treating relatively short vessel lengths, e.g., 0.5-4 cm. The support structure may have different structures.

In one embodiment, the support structure is balloon-expandable from a small diameter state to an expanded final diameter state as a result of expansion beyond the elastic limit of the material forming the structure. Accordingly, such a structure should be formed of material which has sufficient strength and elasticity to be expanded and to retain its expanded diameter, e.g., silver, tantalum, stainless steel, gold, titanium, and plastic. Such a structure may be formed of a plurality of intersecting elongate members, which are fixedly secured to one another by welding, soldering, or gluing.

In another embodiment, the support structure is self-expanding. Such a support structure has a natural state of a preselected large diameter, which is compressed into a small diameter state and inserted into radially pre-dilated tube 50 to form the endovascular stent. The support structure is formed from shape-memory material, e.g., nitinol (nickel titanium alloy), which tends to expanded to its original preselected diameter.

In yet another embodiment, the support structure is formed from thermally-activated shape memory alloy (e.g., nitinol), which expands to a preselected implantation diameter state at temperatures above a threshold temperature (selected to be below normal body temperature) and is pliable and non-expanding a lower temperatures.

Referring to Figs. 3 and 3A, a stent 54 includes an elongated radially pre-dilated PTFE tube 51 disposed about two support structures 56, 58 located at opposite ends of the tube. For vascular applications, the PTFE tube is generally 5-45 cm long and the support structures are about 1-2 cm long. Stent 54 is useful for supporting or sealing relatively long lengths of body passages. The support structures may be formed in accordance with the structures described above in connection with Figs. 2 and 2A.

As shown in Figs. 4 and 4A, a stent 60 may include more than two support structures disposed inside a radially pre-dilated PTFE tube. In the embodiment shown, there are three such support structures. Stent 60 is useful in areas of the vascular anatomy where there are bends or other anatomical features and in which an additional support prevents collapse of the lumen defined through the stent. The support structures may be formed in accordance with the structures described above in connection with Figs. 2 and 2A.

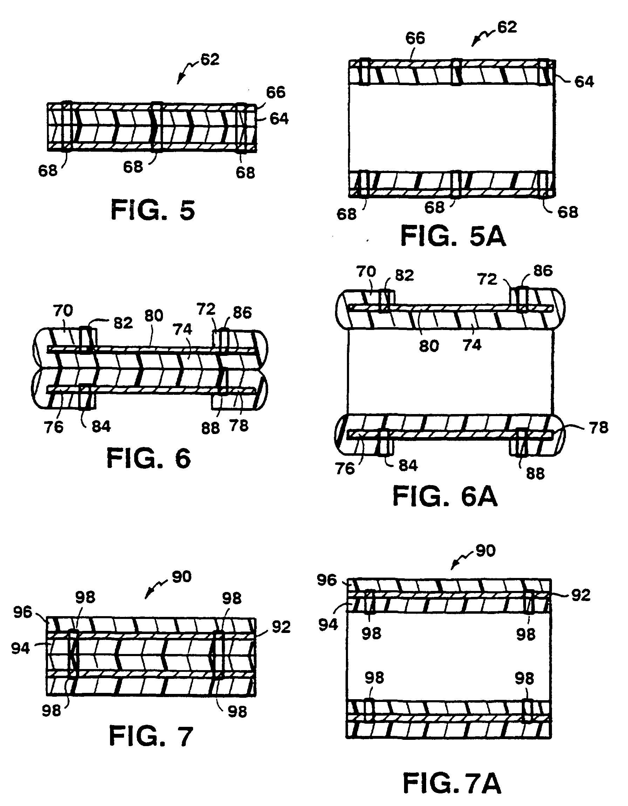

Referring to Figs. 5 and 5A, a stent 62 includes a tubular liner 64 formed from radially pre-dilated PTFE disposed inside a support structure 66. A plurality of sutures or metal clips 68 fix the PTFE liner 64 to the radially expandable support structure 66. There is preferably a set of four clips disposed around the circumference the stent, at a plurality of spaced-apart locations along the length of the stent. The support structure may be formed in accordance with the structures described above in connection with Figs. 2 and 2A.

In the embodiment shown in Figs. 6 and 6A, the ends 70, 72 of a tubular liner 74 extend beyond and wrap back over the exterior of ends 76, 78 of a support structure 80. Sutures 82-88 secure the ends of the tubular liner to the support structure. The support structure may be formed in accordance with the structures described above in connection with Figs. 2 and 2A. In an alternative embodiment, support structure 80 is replaced by a plurality of spaced-apart support structures, with two support structures disposed at the ends thereof.

Referring to Figs. 7 and 7A, in another embodiment, a stent 90 includes a support structure 92, formed in accordance with one of the embodiments described in connection with Figs. 2 and 2A, surrounded by a liner 94 and a cover 96, each formed from radially pre-dilated PTFE tubes. Liner 94 is attached to the support structure by a plurality of sutures or clips 98. The support structure may be formed in accordance with the structures described above in connection with Figs. 2 and 2A. In an alternative embodiment, support structure 92 is replaced by a plurality of spaced-apart support structures, with two support structures disposed at the ends thereof.

Other embodiments are within the scope of the claims. For example, the radially expanded pre-dilated PTFE tubular implants may be coated with a pharmacological agent, such as heparin or antitumor agents.

Still other embodiments are within the scope of the claims.

## Claims

1. A method for producing a radially expandable tube-form medical implant (11) formed of extruded crystalline polytetrafluoroethylene having a microstructure of nodes interconnected by fibrils, comprising the steps of:

   extruding a lubricant/polytetrafluoroethylene resin blend to form a tube-form medical implant (11) having a longitudinal axis, a primary inner diameter, and a primary length;
   heating the tube-form medical implant (11) to remove the lubricant;
   stretching the tube-form medical implant (11) along the longitudinal axis to produce an elongated implant (11) having a secondary length greater than the primary length;
   radially pre-dilating the tube-form medical implant (11) from said primary inner diameter to an expanded diameter that is at least two times said primary inner diameter; and
   sintering the tube-form medical implant (11) to contract the implant from said expanded diameter to a contracted diameter **characterised in that** the contracted diameter is substantially the same as said primary inner diameter, resulting in said implant exhibiting a radial expansion ratio of 1.0,

   wherein the radial expansion ratio is the radial expansion coefficient divided by the radial expansion limit, the radial expansion coefficient being the maximum inflation pressure of the tube-form medical implant without loss of structural integrity divided by the percentage radial dilatation of the implant at the maximum inflation pressure and the radial expansion limit being the pressure of the implant at which it bursts divided by the percentage radial dilatation of the implant at the burst pressure.

2. A method according to Claim 1, wherein the tube-form medical implant (11) is radially pre-dilated to an expanded diameter that is at least three times said primary inner diameter.

**3.** A method according to Claim 2, wherein the tube-form medical implant (11) is radially pre-dilated to an expanded diameter that is at least four times said primary inner diameter.

**4.** A method according to Claim 3, wherein the tube-form medical implant (11) is radially pre-dilated to an expanded diameter that is at least five times said primary inner diameter.

**5.** A method according to any one of Claims 1 to 4 further comprising the step of, after stretching the tube-form medical implant and prior to pre-dilating the tube-form medical implant:

sintering the elongated implant to produce a sintered tube-form medical implant.

**6.** A method according to any one of the preceding claims further comprising the step of, after stretching the tube-form medical implant: restraining the elongate implant (11) to prevent the elongate implant from longitudinally contracting during sintering.

**7.** A method according to Claim 6 wherein the sintered implant (11) produced after the restraining step has a length substantially equivalent to the secondary length.

**8.** A method according to any one of the preceding claims further comprising, after sintering the tube-form medical implant to contract the implant: longitudinally stretching the sintered radially expanding implant (11).

**9.** A method according to any one of the preceding claims further comprising the step of, between pre-dilating the tube-form medical implant and sintering the implant to contract the implant: restraining the ends of the radially expanded implant (11) to maintain the length of the radially expanded implant when the radially expanded implant is sintered.

**10.** An expandable tubular medical implant (11) comprising porous material of crystalline polytetrafluoroethylene polymer having a microstructure of nodes interconnected by fibrils, the tubular implant being permanently, radially expandable from a small delivery diameter, which is of low profile suitable for delivery to an implantation site inside a blood vessel, to a more than 50% larger implantation diameter, which is suitable for implantation inside a blood vessel, by application of outward radial force, the tubular medical implant (11), after expansion to the implantation diameter, substantially retaining its expanded size, tensile strength, and structural integrity so that increase in the outward radial force is required before further radial expansion can occur, **characterised in that** the expandable tubular medical implant (11) exhibits a radial expansion ratio of 1.0, wherein the radial expansion ratio is the radial expansion coefficient divided by the radial expansion limit, the radial expansion coefficient being the maximum inflation pressure of the tubular medical implant (11) without loss of structural integrity divided by the percentage radial dilatation of the implant at the maximum inflation pressure and the radial expansion limit being the pressure of the implant (11) at which it bursts divided by the percentage radial dilatation of the implant (11) at the burst pressure.

**11.** An expandable tubular medical implant according to Claim 10 in combination with an endovascular stent (48, 54, 60, 62,90).

**12.** An expandable tubular medical implant according to Claim 10 or 11 **characterised in that** it is in the form of a liner (64,74,94) for an endovascular stent.

**13.** An expandable tubular medical implant according to Claim 10 or 11 **characterised in that** it is in the form of a cover (50,96) for an endovascular stent.

**14.** An expandable tubular medical implant according to Claim 10 to 13 **characterised in that** it has the characteristic of being expandable by an angioplasty balloon catheter at a pressure of 507 to 1520 kPa (5 to 15 atmospheres).

**15.** An expandable tubular medical implant according to Claim 10 to 14 **characterised in that** it has the characteristic that its longitudinal length does not substantially change as a result of radial expansion.

**16.** An expandable endovascular stent (48, 54, 60, 62, 90) having a contracted diameter state with an outer diameter suitable for delivery through the vasculature of a patient to a desired site and an expanded diameter state with an outer diameter suitable for supporting a blood vessel at the desired site, being **characterised in that** it comprises

an expandable tubular medical implant (50,51,64, 74, 94,96) according to any one of Claims 10 to 15 having an interior wall surface and an exterior wall surface, and

at least one tubular, radially expandable support structure (52, 56,58,66,80,92) coupled to the tubular medical implant, the support structure having an interior wall surface and an exterior wall surface.

17. A stent according to Claim 16 **characterised in that** the expandable tubular medical implant is disposed on the exterior wall surface of the at least one support structure (52, 56, 58, 92).

18. A stent according to Claim 16 or Claim 17 **characterised in that** the expandable tubular medical implant has first and second ends, there being two of the support structures disposed within the interior wall surface of the medical implant at first and second ends thereof, respectively.

19. A stent according to Claim 16 **characterised in that** the expandable tubular medical implant is disposed within the interior wall surface of the at least one support structure (66,80,92).

20. A stent according to Claim 19 **characterised in that** an extension of the expandable tubular medical implant wraps around an end (76,78) of the at least one support structure (80), from the interior wall surface to the exterior wall surface of the support structure.

21. A stent according to Claim 16 **characterised in that** the first expandable tubular medical implant (94) is disposed within the interior wall surface of the at least one support structure (92), and further comprising a second expandable tubular medical implant (96) according to any one of Claims 10 to 15 disposed about the exterior wall surface of the at least one support structure.

22. A stent according to any one of Claims 16 to 21 **characterised in that** the at least one radially expandable support structure (52,56,58,66,80,92) retains its shape upon expansion to the expanded diameter state as a result of deformation beyond the elastic limit of the material forming the support structure.

23. A stent according to any one of Claims 16 to 22 **characterised in that** the at least one or more radially expandable support structure is radially self-expandable to the expanded diameter state.

24. A stent according to Claim 23 **characterised in that** the at least one radially expandable support structure is self-expanding as a result of thermal activation.

25. A stent according to any one of Claims 16 to 24 **characterised in that** the at least one support structure is formed from stainless steel or nitinol.

26. A stent according to any one of Claims 16 to 25 **characterised in that** the expandable tubular medical implant is coated with a pharmacological agent.

**Patentansprüche**

1. Verfahren zur Herstellung eines radial expandierbaren röhrenförmigen medizinischen Implantates (11), geformt aus extrudiertem kristallinem Polytetrafluorethylen mit einer Mikrostruktur von Knoten, die durch Fibrillen miteinander verbunden sind, umfassend die Schritte des:

Extrudierens einer Gleitmittel/Polytetrafluorethylenharz-Mischung, um ein röhrenförmiges medizinisches Implantat (11) mit einer Längsachse, einem primären Innendurchmesser und einer primären Länge zu formen;

Erwärmens des röhrenförmigen medizinischen Implantates (11), um das Gleitmittel zu entfernen;

Streckens des röhrenförmigen medizinischen Implantates (11) entlang der Längsachse, um ein gestecktes Implantat (11) mit einer sekundaren Länge, die größer ist als die primären Länge, zu erzeugen;

radialen Prädilatierens des röhrenförmigen medizinischen Implantates (11) von dem primären Innendurchmesser zu einem expandierten Durchmesser, der mindestens das zweifache des primären Innendurchmessers beträgt; und

Sinterns des röhrenförmigen medizinischen Implantates (11), um das Implantat vom expandierten Durchmesser zu einem kontrahierten Durchmesser zu kontrahieren, **dadurch gekennzeichnet, dass** der kontrahierte Durchmesser im Wesentlichen derselbe ist wie der primäre Innendurchmesser, wobei das Ergebnis das Implantat ist, das ein radiales Expansionsverhältnis von 1,0 zeigt,

wobei das radiale Expansionsverhältnis der radiale Ausdehnungskoeffizient, geteilt durch die radiale Expansionsgrenze, ist, wobei der radiale Ausdehnungskoeffizient der maximale Aufblasdruck des röhrenförmigen medizinischen Implantates ohne Verlust der strukturellen Integrität, geteilt durch die radiale Dilatation des Implantates in Prozent beim maximalen Aufblasdruck, ist, und wobei die radiale Expansionsgrenze der Druck des Implantates, bei welchem es birst, geteilt durch die radiale Dilatation des Implantates in Prozent beim Berstdruck, ist.

**2.** Verfahren gemäß Anspruch 1, wobei das röhrenförmige medizinische Implantat (11) zu einem expandierten Durchmesser radial prädilatiert wird, der mindestens das Dreifache des primären Innendurchmessers beträgt.

**3.** Verfahren gemäß Anspruch 2, wobei das röhrenförmige medizinische Implantat (11) zu einem expandierten Durchmesser radial prädilatiert wird, der mindestens das Vierfache des primären Innendurchmessers beträgt.

**4.** Verfahren gemäß Anspruch 3, wobei das röhrenförmige medizinische Implantat (11) zu einem expandierten Durchmesser radial prädilatiert wird, der mindestens das Fünffache des primären Innendurchmessers beträgt.

**5.** Verfahren gemäß einem der Ansprüche 1 bis 4, ferner umfassend, nach dem Strecken des röhrenförmigen medizinischen Implantates und vor dem Prädilatieren des röhrenförmigen medizinischen Implantates, den Schritt des:

Sinterns des gestreckten Implantates, um ein gesintertes röhrenförmiges medizinisches Implantat zu erzeugen.

**6.** Verfahren gemäß einem der vorangegangenen Ansprüche, ferner umfassend, nach dem Strecken des röhrenförmigen medizinischen Implantates, den Schritt des:

Einspannens des gestreckten Implantates (11), um das gestreckte Implantat daran zu hindern, während des Sinterns longitudinal zu kontrahieren.

**7.** Verfahren gemäß Anspruch 6, wobei das gesinterte Implantat (11), das nach dem Einspannschritt erzeugt worden ist, eine Länge aufweist, die im Wesentlichen gleich der sekundären Länge ist.

**8.** Verfahren gemäß einem der vorangegangenen Ansprüche, ferner umfassend, nach dem Sintern des röhrenförmigen medizinischen Implantates, um das Implantat zu kontrahieren: longitudinales Strecken des gesinterten radial expandierenden Implantates (11).

**9.** Verfahren gemäß einem der vorangegangenen Ansprüche, ferner umfassend, zwischen dem Prädilatieren des röhrenförmigen medizinischen Implantates und dem Sintern des Implantates, um das Implantat zu kontrahieren, den Schritt des: Einspannens der Enden des radial expandierten Implantates (11), um die Länge des radial expandierten Implantates aufrechtzuerhalten, wenn das radial expandierte Implantat gesintert wird.

**10.** Expandierbares röhrenförmiges medizinisches Implantat (11), umfassend poröses Material von kristallinem Polytetrafluorethylen-Polymer mit einer Mikrostruktur von Knoten, die durch Fibrillen miteinander verbunden sind, wobei das röhrenförmige Implantat von einem kleinen Zuführdurchmesser, welcher von geringem Profil ist, geeignet zur Zuführung zu einer Implantationsstelle im Innern eines Blutgefäßes, zu einem mehr als 50 % größeren Implantationsdurchmesser, welcher zur Implantation im Innern eines Blutgefäßes geeignet ist, durch Anwenden einer äußeren radialen Kraft permanent radial expandierbar ist, wobei das röhrenförmige medizinische Implantat (11), nach Expansion zum Implantationsdurchmesser, seine expandierte Größe, Zugfestigkeit und strukturelle Integrität im wesentlichen beibehält, so dass eine Zunahme der äußeren radialen Kraft erforderlich ist, bevor weitere radiale Expansion stattfinden kann, **dadurch gekennzeichnet, dass** das expandierbare röhrenförmige medizinische Implantat (11) ein radiales Expansionsverhältnis von 1,0 zeigt, wobei das radiale Expansionsverhältnis der radiale Ausdehnungskoeffizient, geteilt durch die radiale Expansionsgrenze, ist, wobei der radiale Ausdehnungskoeffizient der maximale Aufblasdruck des röhrenförmigen medizinischen Implantates (11) ohne Verfust der strukturellen Integrität, geteilt durch die radiale Dilatation des Implantates in Prozent beim maximalen Aufblasdruck, ist, und wobei die radiale Expansionsgrenze der Druck des Implantates (11), bei welchem es birst, geteilt durch die radiale Dilatation des Implantates (11) in Prozent beim Berstdruck, ist.

11. Expandierbares röhrenförmiges medizinisches Implantat gemäß Anspruch 10 in Verbindung mit einem Endovaskularstent (48, 54, 60, 62, 90).

12. Expandierbares röhrenförmiges medizinisches Implantat gemäß Anspruch 10 oder 11, **dadurch gekennzeichnet, dass** es in Form einer Auskleidung (64, 74, 94) für einen Endovaskularstent ist.

13. Expandierbares röhrenförmiges medizinisches Implantat gemäß Anspruch 10 oder 11, **dadurch gekennzeichnet, dass** es in Form einer Umhüllung (50, 96) für einen Endovaskularstent ist.

14. Expandierbares röhrenförmiges medizinisches Implantat gemäß Anspruch 10 bis 13, **dadurch gekennzeichnet, dass** es die Eigenschaft aufweist, durch einen Angioplastie-Ballonkatheter wie bei einem Druck von 507 bis 1520 kPa (5 bis 15 Atmosphären) expandierbar zu sein.

15. Expandierbares röhrenförmiges medizinisches Implantat gemäß Anspruch 10 bis 14, **dadurch gekennzeichnet, dass** es die Eigenschaft aufweist, dass es seine longitudinale Länge als Ergebnis radialer Expansion im Wesentlichen nicht verändert.

16. Expandierbarer Endovaskularstent (48, 54, 60, 62, 90) im Zustand kontrahierten Durchmessers mit einem Außendurchmesser, der zur Zuführung durch das Gefäßsystem eines Patienten zu einer gewünschten Stelle geeignet ist, und im Zustand expandierten Durchmessers mit einem Außendurchmesser, der zur Unterstützung eines Blutgefäßes an der gewünschten Stelle geeignet ist, **dadurch gekennzeichnet, dass** er
ein expandierbares röhrenförmiges medizinisches Implantat (50, 51, 64, 74, 94, 96) gemäß einem der Ansprüche 10 bis 15 mit einer Innenwandfläche und einer Außenwandfläche, und
mindestens eine röhrenförmige, radial expandierbare Stützstruktur (52, 56, 58, 66, 80, 92) umfasst, die mit dem röhrenförmigen medizinischen Implantat verbunden ist, wobei die Stützstruktur eine Innenwandfläche und eine Außenwandfläche aufweist.

17. Stent gemäß Anspruch 16, **dadurch gekennzeichnet, dass** das expandierbare röhrenförmige medizinische Implantat auf der Außenwandfläche der mindestens einen Stützstruktur (52, 56, 58, 92) angeordnet ist.

18. Stent gemäß Anspruch 16 oder Anspruch 17, **dadurch gekennzeichnet, dass** das expandierbare röhrenförmige medizinische Implantat erste und zweite Enden aufweist, wobei dort zwei der Stützstrukturen innerhalb der Innenwandfläche des medizinischen Implantates an den ersten beziehungsweise zweiten Enden davon angeordnet sind.

19. Stent gemäß Anspruch 16, **dadurch gekennzeichnet, dass** das expandierbare röhrenförmige medizinische Implantat innerhalb der Innenwandfläche der mindestens einen Stützstruktur (66, 80, 92) angeordnet ist.

20. Stent gemäß Anspruch 19, **dadurch gekennzeichnet**, das eine Extension des expandierbaren röhrenförmigen medizinischen Implantates ein Ende (76, 78) der mindestens einen Stützstruktur (80) von der Innenwandfläche zur Außenwandfläche der Stützstruktur umhüllt.

21. Stent gemäß Anspruch 16, **dadurch gekennzeichnet, dass** das erste expandierbare röhrenförmige medizinische Implantat (94) innerhalb der Innenwandfläche der mindestens einen Stützstruktur (92) angeordnet ist, und der ferner ein zweites expandierbares röhrenförmiges medizinisches Implantat (96) gemäß einem der Ansprüche 10 bis 15 umfasst, das um die Außenwandfläche der mindestens einen Stützstruktur angeordnet ist.

22. Stent gemäß einem der Ansprüche 16 bis 21, **dadurch gekennzeichnet, dass** die mindestens eine radial expandierbare Stützstruktur (52, 56, 58, 66, 80, 92) ihre Form bei Expansion zum Zustand expandierten Durchmessers als Ergebnis von Verformung jenseits der Elastizitätsgrenze des Materials, das die Stützstruktur bildet, beibehält.

23. Stent gemäß einem der Ansprüche 16 bis 22, **dadurch gekennzeichnet, dass** die mindestens eine oder mehrere radial expandierbare Stützstruktur zum Zustand expandierten Durchmessers radial selbst-expandierbar ist.

**24.** Stent gemäß Anspruch 23, **dadurch gekennzeichnet, dass** die mindestens eine radial expandierbare Stützstruktur als Ergebnis thermischer Aktivierung selbst-expandierbar ist.

**25.** Stent gemäß einem der Ansprüche 16 bis 24, **dadurch gekennzeichnet, dass** die mindestens eine Stützstruktur aus rostfreiem Stahl oder Nitinol geformt ist.

**26.** Stent gemäß einem der Ansprüche 16 bis 25, **dadurch gekennzeichnet, dass** das expandierbare röhrenförmige medizinische Implantat mit einem pharmakologischen Mittel bedeckt ist.

**Revendications**

**1.** Procédé de production d'un implant médical en forme de tube (11), expansible radialement, formé d'un polytétrafluoroéthylène cristallin extrudé, ayant une microstructure de noeuds interconnectés par des fibrilles, comprenant les étapes de :

extrusion d'une mélange lubrifiant/résine polytétrafluoroéthylène pour former un implant médical en forme de tube (11) ayant un axe longitudinal, un diamètre interne primaire et une longueur primaire ;

chauffage de l'implant médical en forme de tube (11) pour éliminer le lubrifiant ;

étirement de l'implant médical en forme de tube (11) le long de l'axe longitudinal pour produire un implant allongé (11) ayant une longueur secondaire supérieure à la longueur primaire ;

prédilatation radiale de l'implant médical en forme de tube (11) depuis ledit diamètre interne primaire jusqu'à un diamètre expansé qui est au moins deux fois celui dudit diamètre primaire ; et

frittage de l'implant médical en forme de tube (11) pour contracter l'implant depuis ledit diamètre expansé en direction d'un diamètre contracté, **caractérisé en ce que** le diamètre contracté est sensiblement le même que celui dudit diamètre interne primaire, donnant ledit implant présentant un rapport d'expansion radiale de 1,0,

dans lequel le rapport d'expansion radiale est le coefficient d'expansion radiale divisé par la limite d'expansion radiale, le coefficient d'expansion radiale étant la pression d'inflation maximale de l'implant médical en forme de tube sans perte d'intégrité structurelle, divisée par le pourcentage de la dilatation radiale de l'implant à la pression d'inflation maximale et la limite d'expansion radiale étant la pression de l'implant à laquelle il éclate, divisée par le pourcentage de dilatation radiale de l'implant à la pression d'éclatement.

**2.** Procédé selon la revendication 1, dans lequel l'implant médical en forme de tube (11) est radialement prédilaté à un diamètre expansé qui est au moins trois fois celui dudit diamètre primaire interne.

**3.** Procédé selon la revendication 2, dans lequel l'implant médical en forme de tube (11) est radialement prédilaté à un diamètre expansé qui est au moins quatre fois celui dudit diamètre primaire interne.

**4.** Procédé selon la revendication 3, dans lequel l'implant médical en forme de tube (11) est radialement prédilaté à un diamètre expansé qui est au moins cinq fois celui dudit diamètre primaire interne.

**5.** Procédé selon l'une quelconque des revendications 1 à 4, comprenant l'étape, après étirement de l'implant médical en forme de tube et avant prédilatation de l'implant médical en forme de tube, de :

frittage de l'implant allongé pour produire un implant médical en forme de tube fritté.

**6.** Procédé selon l'une quelconque des revendications précédentes, comprenant en outre l'étape, après étirement de l'implant médical en forme de tube et avant prédilatation de l'implant médical en forme de tube, de :

restriction de l'implant allongé (11) pour éviter que l'implant allongé ne se contracte longitudinalement pendant le frittage.

**7.** Procédé selon la revendication 6, dans lequel l'implant fritté (11) produit après l'étape de restriction a une longueur sensiblement équivalente à la longueur secondaire.

**8.** Procédé selon l'une quelconque des revendications précédentes comprenant en outre, après frittage de l'implant médical en forme de tube pour contracter l'implant : étirement longitudinal de l'implant radialement expansé fritté (11).

**9.** Procédé selon l'une quelconque des revendications précédentes comprenant en outre l'étape, entre la prédilatation de l'implant médical en forme de tube et le frittage de l'implant pour contracter l'implant, de restriction des extrémités de l'implant expansé radialement (11) pour maintenir la longueur de l'implant radialement expansé quand l'implant radialement expansé est fritté.

**10.** Implant médical tubulaire expansible (11), comprenant un matériau poreux du polymère de polytétrafluoroéthylène cristallin ayant une microstructure de noeuds interconnectés par des fibrilles, l'implant tubulaire étant expansible radialement de manière permanente, depuis un petit diamètre de distribution, qui est de profil bas approprié à la distribution sur un site d'implantation à l'intérieur d'un vaisseau sanguin, à un diamètre d'implantation plus large de plus de 50 %, qui est approprié à l'implantation à l'intérieur d'un vaisseau sanguin, par l'application d'une force radiale vers l'extérieur, l'implant médical tubulaire (11), après expansion vers le diamètre de l'implantation, retenant substantiellement sa taille expansée, résistance à la traction et intégrité structurelle de sorte que l'augmentation de force radiale vers l'extérieur est requise avant qu'une autre expansion radiale ne se produise, **caractérisé en ce que** l'implant médical tubulaire expansible (11) présente un rapport d'expansion radiale de 1,0, où le rapport d'expansion radiale est le coefficient d'expansion radiale divisé par la limite d'expansion radiale, le coefficient d'expansion radiale étant la pression d'inflation maximale de l'implant médical tubulaire (11) sans perte d'intégrité structurelle, divisée par le pourcentage de la dilatation radiale de l'implant à la pression d'inflation maximale et la limite d'expansion radiale étant la pression de l'implant (11) à laquelle il éclate, divisée par le pourcentage de dilatation radiale de l'implant (11) à la pression d'éclatement.

**11.** Implant médical tubulaire expansible selon la revendication 10, combiné à une endoprothèse vasculaire (48, 54, 60, 62, 90).

**12.** Implant médical tubulaire expansible selon la revendication 10 ou 11, **caractérisé en ce qu'**il se présente sous la forme d'un habillage (64, 74, 94) pour une endoprothèse vasculaire.

**13.** Implant médical tubulaire expansible selon la revendication 10 ou 11, **caractérisé en ce qu'**il se présente sous la forme d'une couverture (50, 96) pour une endoprothèse vasculaire.

**14.** Implant médical tubulaire expansible selon la revendication 10 à 13, **caractérisé en ce qu'**il a la caractéristique d'être expansible par un cathéter ballon d'angioplastie à une pression de 507 à 1520 kPa (5 à 15 atmosphères).

**15.** Implant médical tubulaire expansible selon la revendication 10 à 14, **caractérisé en ce qu'**il a pour caractéristique que sa longueur longitudinale ne change pas substantiellement à la suite de l'expansion radiale.

**16.** Endoprothèse vasculaire expansible (48, 54, 60, 62, 90) ayant un état de diamètre contracté avec un diamètre externe approprié à la distribution à travers le système vasculaire d'un patient en direction d'un site désiré et un état de diamètre expansé avec un diamètre externe approprié à supporter un vaisseau sanguin sur le site désiré, **caractérisée en ce qu'**elle comprend :

un implant médical tubulaire expansible (50, 51, 64, 74, 94, 96) selon l'une quelconque des revendications 10 à 15 ayant une surface de paroi intérieure et une surface de paroi extérieure, et

au moins une structure support expansible radialement tubulaire (52, 56, 58, 66, 80, 92) couplée à l'implant médical tubulaire, la structure support ayant une surface de paroi intérieure et une surface de paroi extérieure.

**17.** Endoprothèse selon la revendication 16, **caractérisée en ce que** l'implant médical tubulaire expansible est disposé sur la surface de la paroi extérieure d'au moins une structure support (52, 56, 58, 92).

**18.** Endoprothèse selon la revendication 16 ou 17, **caractérisée en ce que** l'implant médical tubulaire expansible possède des première et seconde extrémités, où deux des structures supports sont disposées dans la surface de la paroi intérieure de l'implant médical aux premières et secondes extrémités de celui-ci respectivement.

19. Endoprothèse selon la revendication 16, **caractérisée en ce que** l'implant médical tubulaire expansible est disposé dans la surface de la paroi intérieure d'au moins une structure support (66, 80, 92).

20. Endoprothèse selon la revendication 19, **caractérisée en ce qu'**une expansion de l'implant médical tubulaire expansible s'enroule autour d'une extrémité (76, 78) d'au moins une structure support (80), depuis la surface de la paroi intérieure vers la surface de la paroi extérieure de la structure support.

21. Endoprothèse selon la revendication 16, **caractérisée en ce que** le premier implant médical tubulaire expansible (94) est disposé dans la surface de la paroi intérieure d'au moins une structure support (92) et comprenant en outre un second implant médical tubulaire expansible (96) selon l'une quelconque des revendications 10 à 15, disposé autour de la surface de la paroi extérieure d'au moins une structure support.

22. Endoprothèse selon l'une quelconque des revendications 16 à 21, **caractérisée en ce qu'**au moins une structure support expansible radialement (52, 56, 58, 66, 80, 92) retient sa forme lors de l'expansion vers un état de diamètre expansé à la suite de la déformation au-delà de la limite élastique du matériau formant la structure support.

23. Endoprothèse selon l'une quelconque des revendications 16 à 22, **caractérisée en ce qu'**au moins une ou plusieurs structures supports radialement expansibles sont radialement auto-expansibles vers un état de diamètre expansé.

24. Endoprothèse selon la revendication 23, **caractérisée en ce qu'**au moins une structure support radialement expansible est auto-expansée comme résultat de l'activation thermique.

25. Endoprothèse selon l'une quelconque des revendications 16 à 24, **caractérisée en ce qu'**au moins une structure support est formée d'acier inoxydable ou de nitinol.

26. Endoprothèse selon l'une quelconque des revendications 16 à 25 **caractérisée en ce que** l'implant médical tubulaire expansible est enduit d'un agent pharmacologique.

FIG. 1

FIG. 2

FIG. 2A

FIG. 3

FIG. 3A

FIG. 4

FIG. 4A

FIG. 5

FIG. 5A

FIG. 6

FIG. 6A

FIG. 7

FIG. 7A